# EUROPEAN PATENT APPLICATION

(11) **EP 3 275 475 A1**
(43) Date of publication of application: **31.01.2018**
(21) Application number: 16768197.2
(22) Date of filing: 09.02.2016
(51) Int. Cl.: A61M 5/142, A61M 5/145

(54) **MEDICAL PUMP, MEDICAL PUMP SYSTEM, AND METHOD OF CONTROLLING MEDICAL PUMP SYSTEM**

(30) Priority: 26.03.2015 JP 2015065011
(71) Applicant: Terumo Kabushiki Kaisha, Tokyo 151-0072 (JP)
(72) Inventor: FUJII Toshihiro, Ashigarakami-gun Kanagawa 259-0151 (JP)
(74) Representative: KIPA AB
(86) International application number: PCT/JP2016/053867
(87) International publication number: WO 2016/152294

(57) **Abstract**

Provided is a medical pump for achieving continuous administration of medicine by performing sequential driving with another medical pump. The medical pump includes: a pump driving unit for administering the medicine in accordance with a setting; a communication unit for performing communication between the other medical pump; a control unit for controlling individual driving of the pump driving unit and the communication unit; and an operation unit connected to the control unit. In a single administration period by a first medical pump in continuous administration of the medicine, the control unit determines timing of starting cooperative administration of simultaneously driving the pump driving unit together with a pump driving unit of the other medical pump on the basis of the setting and on the basis of an input from the operation unit.

## Description

### BACKGROUND

### Technical Field

The present invention relates to a medical pump, a medical pump system, and a method for controlling the medical pump system and particularly relates to a medical pump, a medical pump system, and a method for controlling the medical pump system, for achieving continuous administration of medicine using two medical pumps.

### Related Art

In the medical field, medical pumps such as infusion pumps and syringe pumps are used as pumps for administering medicines into the body of a patient. Generally, these medical pumps have an upper limit on the amount of medicine that can be administered by one pump. Therefore, in order to administer the same type of medicine continuously and in large quantities to a patient, there is a commonly implemented method of preparing two medical pumps and operating these continuously (hereinafter referred to as "W method").

In a medical pump for performing administration of medicine by the W method, liquid delivery from a second pump (succeeding pump) is started slightly before the liquid delivery from a first pump (preceding pump) is finished, thereby avoiding a flow rate fluctuation due to delays in startup of the liquid delivery by the succeeding pump. At this time, the flow rate of each of the two pumps is changed stepwise (in steps) such that the flow rate of the succeeding pump is adjusted to the flow rate of a cooperation starting time point at the time point when the preceding pump completely finishes liquid delivery. It is assumed that the number of steps, the time interval of steps, and the flow rate (step flow rate) in each of the steps are stored as a W method table beforehand in each of the medical pumps in association with medicine information (refer to JP 2013-192890 A below, for example).

### SUMMARY

Meanwhile, in continuous administration of medicine with application of the above-mentioned W method, management of the flow rate of medicine is extremely strict in some cases, leading to a demand for monitoring switching pump operation at the start of cooperative administration of simultaneously driving two pumps. However, in the above-described medical pump, switching operation of two pumps is automatically performed without user intervention. This makes it difficult to register the period of time of execution of the switching operation of the two pumps, leading to a difficulty in efficiently monitoring the switching operation.

In view of the above, the present invention is intended to provide a medical pump, a medical pump system, and a method for controlling the medical pump system capable of monitoring switching operation of pumps as necessary in a cooperative administration period of simultaneously driving two pumps in implementation of continuous administration of medicine by two medical pumps.

A medical pump for achieving this intention according to an embodiment of the present invention is a medical pump for achieving continuous administration of medicine by performing sequential driving with another medical pump, the medical pump including: a pump driving unit for administering the medicine in accordance with a setting; a communication unit for performing communication with the other medical pump; a control unit for controlling individual driving of the pump driving unit and the communication unit; and an operation unit connected to the control unit, in which, in a single administration period by a first medical pump in continuous administration of the medicine, the control unit determines timing of starting cooperative administration of simultaneously driving the pump driving unit together with a pump driving unit of the other medical pump on the basis of the setting and on the basis of a manual input from the operation unit.

Moreover, the medical pump system according to an embodiment of the present invention includes two medical pumps for achieving continuous administration of medicine by sequential driving; a control unit configured to control the continuous administration of the medicine by the two medical pumps; and an operation unit connected to the control unit, in which each of the medical pumps includes: a pump driving unit for administering medicine in accordance with a setting; and a communication unit for performing communication with the outside, and the control unit determines, in a single administration period by a first medical pump among the second medical pumps, timing of starting cooperative administration of simultaneously driving the pump driving units of the two medical pumps on the basis of the setting and on the basis of a manual input from the operation unit.

Furthermore, a method for controlling a medical pump system according to an embodiment of the present invention is a method for controlling a medical pump system configured to achieve continuous administration of medicine by sequentially driving two medical pumps on the basis of a setting, in which timing of starting cooperative administration of simultaneously driving the pump driving units of the two medical pumps during a single administration period of a first medical pump among the two medical pumps is determined in accordance with the setting and in accordance with an arbitrary input from the outside.

According to an embodiment of the present invention described above, it is possible to arbitrarily start cooperative administration of simultaneously driving two pumps by the setting and the input by a medical practitioner, an administrator, or the like, being qualified to operate the medical pump, leading to achieving efficient monitoring of the switching operation of the two pumps.

### BRIEF DESCRIPTION OF DRAWINGS

FIG. 1 is a diagram illustrating a configuration of a medical pump and a medical pump system according to an embodiment;
FIG. 2 is a front view of a medical pump of an embodiment;
FIG. 3 is a diagram for illustrating continuous administration of medicine;
FIG. 4 is a diagram illustrating a display screen of start information of cooperative administration of simultaneously driving two medical pumps;
FIG. 5 is a flowchart (preceding pump side) illustrating continuous administration of medicine in a medical pump and a medical pump system according to an embodiment; and
FIG. 6 is a flowchart (succeeding pump side) illustrating continuous administration of medicine in a medical pump and a medical pump system according to an embodiment.

### DETAILED DESCRIPTION

Embodiments related to a medical pump, a medical pump system, and a method for controlling the medical pump system with application of the present invention will be described in detail below with reference to the drawings.

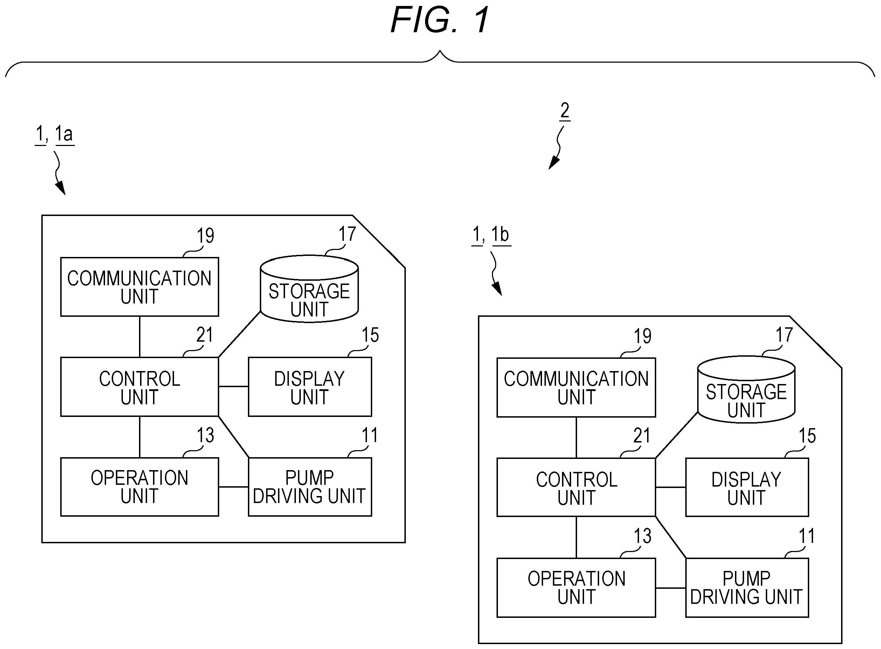

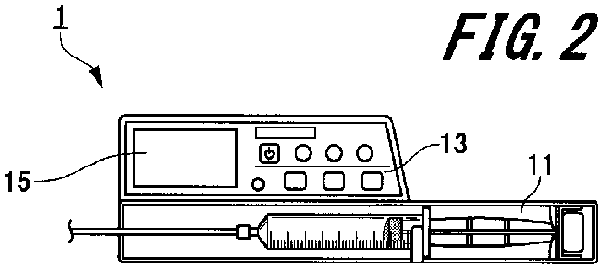

FIG. 1 is a diagram illustrating a configuration of a medical pump 1 and a medical pump system 2 according to an embodiment, and FIG. 2 is a front view of the medical pump 1 according to an embodiment. The medical pump 1 and the medical pump system 2 illustrated in the figures are provided for achieving continuous administration of medicine using two medical pumps 1. Prior to describing the details of the configuration of the medical pump 1 and the medical pump system 2, details of continuous administration of medicine will be described.

### <<Continuous Administration of Medicine>>

Herein, continuous administration of medicine is a medicine administration method of sequentially driving two medical pumps 1, namely, a preceding pump 1 a driven as a first pump, and a succeeding pump 1 b driven as a second pump. Note that the continuous administration of the medicine in this context in particular represents continuous administration using a W method, that is, a method having a period of "cooperative administration" of simultaneously driving two pumps in cooperation at the time of switching between the preceding pump 1 a and the succeeding pump 1 b.

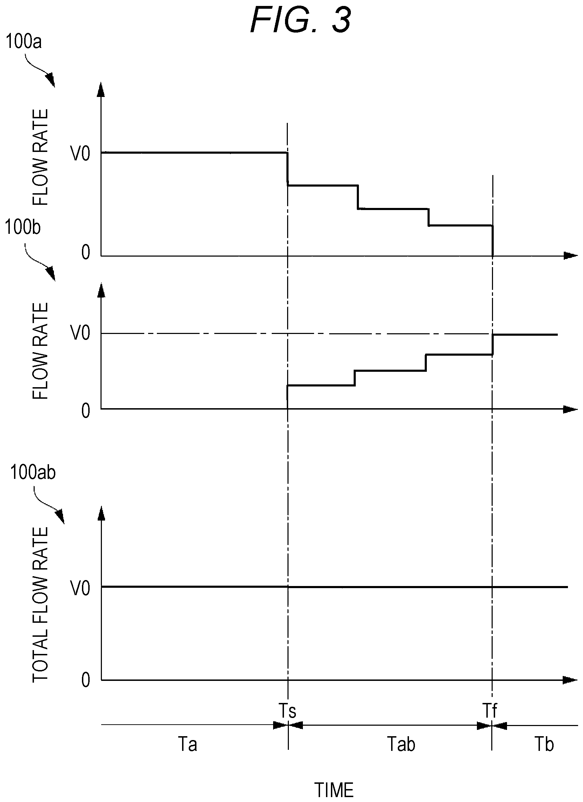

FIG. 3 is a diagram for illustrating continuous administration of medicine with application of this W method, including graphs indicating chronological states of a flow rate 100a of the preceding pump 1 a, a flow rate 100b of the succeeding pump 1 b, and a total flow rate 100ab thereof.

As illustrated in the figures, in a period Ta immediately after the start of administration in continuous administration of the medicine with application of the W method, the flow rate 100a of the preceding pump 1 a is a reference flow rate [V0], the flow rate 100b of the succeeding pump 1 b is [0], and the total flow rate 100ab is the reference flow rate [V0]. This period Ta is a period of driving the preceding pump 1 a alone, and this corresponds to the period Ta for the preceding single administration. While the flow rate 100a of the preceding pump 1 a during the period Ta for the preceding single administration does not need to be constant, the value of the flow rate 100a immediately before the start of the next period is the reference flow rate [V0].

In the period Tab next to the period Ta for the preceding single administration, the flow rate 100a of the preceding pump 1 a decreases stepwise while the flow rate 100b of the succeeding pump 1 b increases stepwise, and the total flow rate 100ab is maintained at the reference flow rate [V0]. This period Tab is a period of simultaneously driving the preceding pump 1 a and the succeeding pump 1 b, and this corresponds to the period Tab for cooperative administration. Note that, herein, the start time of the period Tab for cooperative administration is defined as a cooperation start time Ts, and the end time of the period Tab for cooperative administration is defined as a cooperation end time Tf.

In the period Tb next to the cooperative administration period Tab, the flow rate 100a of the preceding pump 1 a is [0], the flow rate 100b of the succeeding pump 1 b is the reference flow rate [V0], and the total flow rate 100ab is the reference flow rate [V0]. This period is a period of driving the succeeding pump 1 b alone, and herein this corresponds to the period Tb for succeeding single administration.

Continuous administration of the medicine with application of the W method as described above makes it possible to suppress the flow rate fluctuation due to a delay in startup of the succeeding pump 1 b at the time of switching the administration from the preceding pump 1 a to the succeeding pump 1 b, leading to achievement of continuous administration of the medicine at a stable flow rate.

### <<Medical Pump 1 and Medical Pump System 2>>

Each of the medical pumps 1 provided for achieving continuous administration of medicine with application of the W method as described above is, for example, an infusion pump or a syringe pump as illustrated and includes a pump driving unit 11, an operation unit 13, a display unit 15, a storage unit 17, a communication unit 19, and a control unit 21. Details of these components are as follows.

### [Pump driving unit 11]

The pump driving unit 11 is a portion driven for administration of medicine in accordance with the setting, and in a case where the medical pump 1 is a syringe pump, this corresponds to a portion that drives a syringe. In a case where the medical pump 1 is an infusion pump, this corresponds to a driving portion for discharging an infusion liquid in an infusion tube connected to an infusion bag.

### [Operation Unit 13]

The operation unit 13 includes, for example, a power operation unit, an operation unit for starting pump operation, a display switching unit for the display unit 15, and a setting operation unit for operating various settings. With an input from this operation unit 13, driving states of the pump driving unit 11 and display details of the display unit 15 are operated, and selection is performed from an option image displayed on the display unit 15. The operation unit 13 may be a touch panel provided on the display unit 15.

### [Display unit 15]

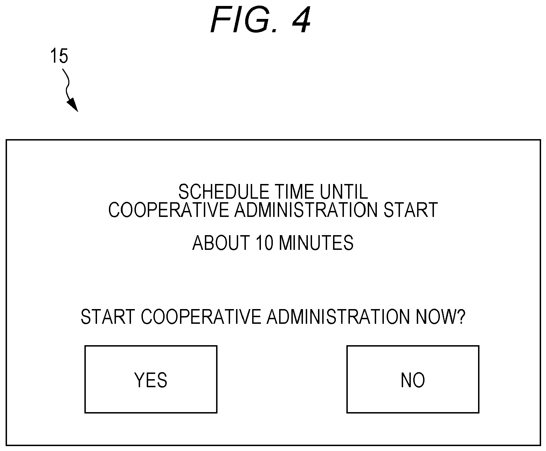

The display unit 15 is provided as an information transmission unit that transmits pump information, represented by a display panel such as a liquid crystal display panel or an organic EL display panel, for example. The display unit 15 displays various types of information in accordance with an instruction from the control unit 21 or an input from the operation unit 13. Examples of the information displayed, as illustrated in FIG. 4, include start information for starting cooperative administration in continuous administration of medicine and scheduled time based on settings up to the cooperation start time Ts, at which cooperative administration is started.

### [Storage Unit 17]

The storage unit 17 stores medicine information related to various medicines administered by the medical pump 1. Herein, in particular, it is assumed that settings related to continuous administration of medicine with application of the W method are stored in association with medicine information, as a W method table. With reference to FIG. 3, the settings related to continuous administration include (1) the number of stages for flow rate change (herein, four stages, for example), (2) the period (time period) of each of the stages (3) flow rate 100a of the preceding pump 1 a and flow rate 100b of the succeeding pump 1 b at each of the stages, in the period Tab of cooperative administration.

Note that (3) flow rate 100a of the preceding pump 1 a and flow rate 100b of the succeeding pump 1 b at each of the stages may be set as a ratio to the reference flow rate [V0] being 100%.

### [Communication Unit 19]

The communication unit 19 is provided for achieving communication between the medical pumps 1 used in the medical pump system 2 and includes a function of transmitting and receiving with infrared rays, for example. The communication unit 19 may also include a communication module function for establishing communication between these medical pumps 1 in a case where two medical pumps 1 for performing continuous administration of medicine are arranged adjacent to each other.

### [Control unit 21]

The control unit 21 is connected to each of the pump driving unit 11, the operation unit 13, the display unit 15, the storage unit 17, and the communication unit 19, and functions as a calculation unit for controlling the drive of the pump driving unit 11, the display unit 15, and the communication unit 19 in accordance with a preset program and operation on the operation unit 13. The control unit 21 is a portion for achieving continuous administration of the medicine with application of the W method and is a portion configured to determine the timing of starting cooperative administration and execute programs for controlling the driving of the pump driving unit 11 of each of the medical pumps 1. The above-configured control unit 21 includes, for example, a central processing unit (CPU), a random access memory (RAM), and a read only memory (ROM), not being illustrated here. Next, details of continuous administration of medicine with application of the W method, to be processed, determined and executed by the control unit 21 will be described.

### <<Control Method of Medical Pump System 2>>

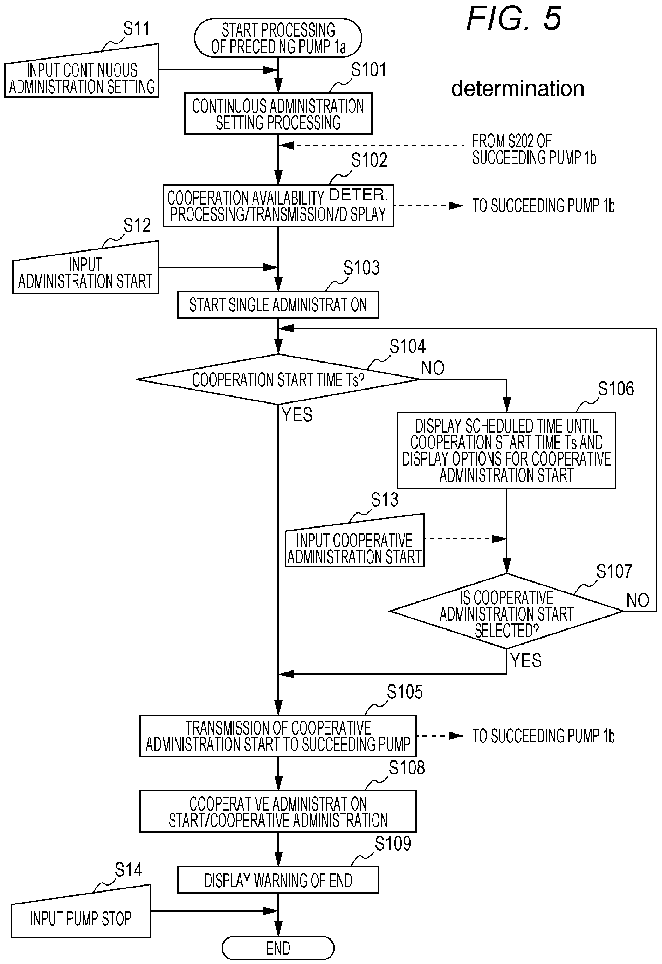

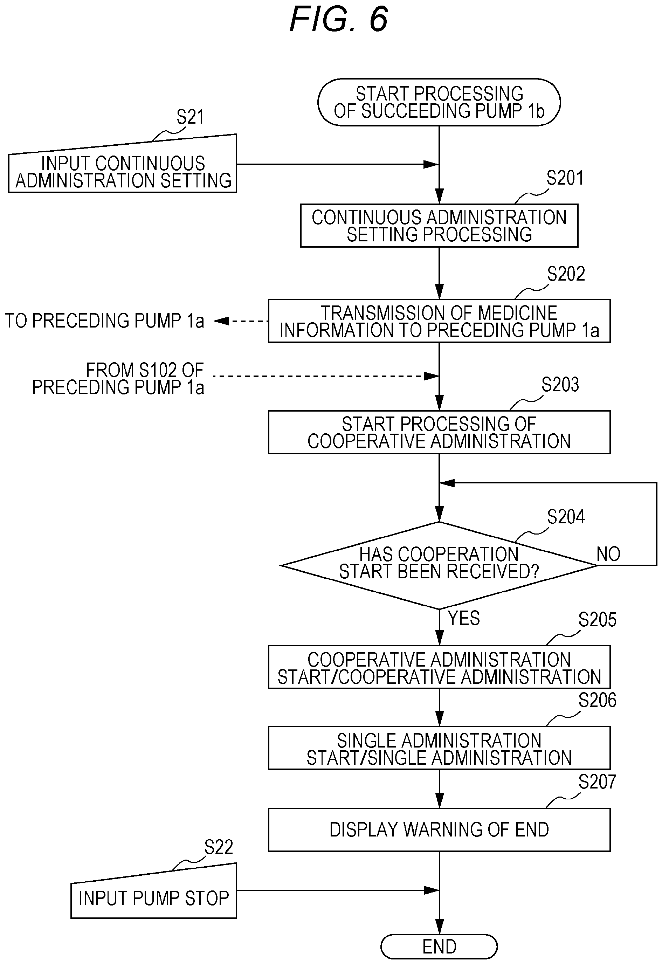

FIGS. 5 and 6 are flowcharts for illustrating a method for controlling the medical pump system 2 performed by the control unit 21 of the medical pump 1 described above, illustrating a control procedure for achieving continuous administration of medicine with application of the W method. Among the figures, FIG. 5 relates to the preceding pump 1a, and FIG. 6 relates to the succeeding pump 1b. These flows are implemented by execution of programs stored in the ROM and RAM by the CPU constituting each of the control units 21 of the preceding pump 1 a and the succeeding pump 1b. Hereinafter, a procedure for controlling the medical pump system 2 performed by the control unit 21 of each of the medical pumps 1 will be described in accordance with the flowcharts of FIGS. 5 and 6 with reference to FIGS. 1 to 4.

First, it is assumed that, as a preliminary stage of executing continuous administration of medicine, processing of establishing communication between two medical pumps 1 used for continuous administration, and processing of setting one of the two as the preceding pump 1a and the other as the succeeding pump 1b have been performed. The processing may be performed as ordinary processing disclosed in the above-mentioned known documents, without a procedure thereof being limited. The two medical pumps 1 may have the same configuration, and any of the medical pumps 1 can either be set as the preceding pump 1a or the succeeding pump 1b.

### [Step S11 (preceding pump 1 a: FIG. 5)]

First, in step S11, a medical practitioner, an administrator, or the like, being qualified to operate the medical pump system 2 inputs settings related to continuous administration of medicine by operating the preceding pump 1a-side operation unit 13. The settings to be input are (a) medicine information and (b) administration dose, regarding continuous administration, and these settings are input by selection with operation on the operation unit 13.

### [Step S101 (preceding pump 1 a: FIG. 5)]

In step S101, the preceding pump 1a-side control unit 21 performs setting processing of continuous administration of medicine with application of the W method. At this point, the control unit 21 reads the setting related to continuous administration corresponding to (a) medicine information selected in step S11, from the W method table stored in each of the storage units 17. The settings read at this point include (1) the number of stages for flow rate change (herein, four stages, for example), (2) the period (time period) of each of the stages, (3) flow rate 100a of the preceding pump 1 a at each of the stages, in the period Tab of cooperative administration, as described above.

In particular, the control unit 21 of the preceding pump 1 a calculates the cooperation start time Ts and the cooperation end time Tf illustrated in FIG. 3 on the basis of (2) the period (time period) of each of the stages that has been read and (b) administration dose set in step S11. With this configuration, as illustrated in FIG. 3, the control unit 21 of the preceding pump 1 a sets a driving sequence of the pump driving unit 11 in continuous administration of the medicine to achieve medicine administration with the flow rate 100a being changed chronologically.

### [Step S21 (succeeding pump 1b: FIG. 6)]

In step S21, similarly to step S11, a medical practitioner, an administrator, or the like, being qualified to operate the medical pump system 2 inputs settings related to medicine administration by inputting from the succeeding pump 1 b-side operation unit 13. The settings to be input are (a) medicine information, and (b) administration dose, regarding continuous administration, and these settings are input by selection with operation on the operation unit 13.

### [Step S201 (succeeding pump 1b: FIG. 6)]

In step S201, similarly to step S101 performed by the preceding pump 1a-side control unit 21, the succeeding pump 1b-side control unit 21 performs setting processing of continuous administration of medicine with application of the W method. Note that, as illustrated in FIG. 3, the control unit 21 of the succeeding pump 1b sets a driving sequence of the pump driving unit 11 in continuous administration of the medicine on and after the cooperation start time Ts so as to achieve medicine administration with the flow rate 100b being changed chronologically.

### [Step S202 (succeeding pump 1b: FIG. 6)]

Next, in step S202, the succeeding pump 1 b-side control unit 21 causes the communication unit 19 to transmit (a) the medicine information input in step S21 toward the preceding pump 1 a side.

### [Step S102 (preceding pump 1 a: FIG. 5)]

In step S102, on the basis of the medicine information transmitted from the succeeding pump 1 b and the medicine information input in step S11, the preceding pump 1a-side control unit 21 performs cooperation availability determination processing of determining whether cooperation with the succeeding pump 1 b is available. Herein, it is determined whether the medicine information transmitted from the succeeding pump 1 b matches with the medicine information set for the preceding pump 1a, and in a case where there is a match, processing of determining that cooperation is available is performed. Subsequently, in a case where it is determined that cooperation is available in this determination processing, the control unit 21 displays a message to the effect that cooperation is available to the display unit 15 and causes the communication unit 19 to notify the succeeding pump 1 b side of the message that cooperation is available.

In contrast, in case it is determined that cooperation is not available in this cooperation availability determination processing, continuous administration of the medicine is stopped.

### [Step S203 (succeeding pump 1 b: FIG. 6)]

In step S203, the succeeding pump 1 b-side control unit 21 starts the cooperative administration processing of the medicine upon receiving the transmission of the message that cooperation is available from the preceding pump 1 a side by the communication unit 19.

### [Step S204 (succeeding pump 1 b: Fig. 6)]

With this processing, in step S204, the succeeding pump 1 b-side control unit 21 waits for reception of the cooperation start transmitted from the preceding pump 1 a side and repeats determination of presence/absence of the reception of cooperation start until the determination of presence of reception (Yes) is performed.

### [Step S12 (preceding pump 1 a: FIG. 5)]

Meanwhile, in step S12, after confirming the cooperation availibility display on the preceding pump 1a-side display unit 15, the medical practitioner, the administrator, or the like, being qualified to operate the medical pump system 2, performs input of administration start by operating the operation unit 13. Herein, for example, continuous administration of the medicine is started by keep pressing the administration start button on the operation unit 13.

### [Step S103 (preceding pump 1 a: FIG. 5)]

Next, in step S103, in response to the input of the administration start, the preceding pump 1a-side control unit 21 drives the pump driving unit 11 in accordance with the driving sequence set in step S101. With this operation, single administration for the preceding single administration period Ta illustrated in FIG. 3 is started.

### [Step S104 (preceding pump 1 a: FIG. 5)]

Thereafter, in step S104, the preceding pump 1 a-side control unit 21 determines whether it is the time of the cooperation start time Ts for starting cooperative administration. Herein, it is determined whether the time from the time point when single administration is started in step S103 has reached the cooperation start time Ts set by the setting processing and calculation in step S101, and in a case where it is determined the time has reached the cooperation start time Ts (Yes), processing proceeds to step S105. In contrast, in a case where it is determined that the cooperation start time Ts has not been reached (No), the processing proceeds to step S106.

### [Step S105 (preceding pump 1 a: FIG. 5)]

In step S105, the preceding pump 1 a-side control unit 21 causes the communication unit 19 to transmit a message to start cooperative administration to the succeeding pump 1 b side.

### [Step S106 (preceding pump 1 a: FIG. 5)]

In contrast, in step S106, the preceding pump 1a-side control unit 21 causes the display unit 15 to display the scheduled time until the cooperation start time Ts and display the start information for starting the cooperative administration as illustrated in FIG. 4. As illustrated in the figure, herein, this start information is a start selection button to select whether to start cooperative administration from now ("YES" or "NO").

### [Step S13 (preceding pump 1 a: FIG. 5)]

Thereafter, in step S13, after confirming the display on the preceding pump 1a-side display unit 15, the medical practitioner, the administrator, or the like, being qualified to operate the medical pump system 2 performs input of cooperative administration start by manually operating the operation unit 13 as necessary. At this point, the scheduled time before the cooperation start time Ts still remains (for example, 10 minutes). In a case, however, where it is desired to start the cooperative administration by simultaneously driving the preceding pump 1 a and the succeeding pump 1 b without waiting for the start time, selection and input of "Yes" among the selection information displayed on the display unit 15 is performed by operation on the operation unit 13.

### [Step S107 (preceding pump 1 a: FIG. 5)]

In step S107, the preceding pump 1 a-side control unit 21 determines whether an input of cooperative administration start has been performed. In a case where it is determined that the input of cooperative administration start has been performed (Yes), processing proceeds to step S105, and the communication unit 19 is made to transmit a message of starting cooperative administration to the succeeding pump 1 b side. In contrast, in a case where it is determined that the input of cooperative administration start has not been performed (No), processing returns to the previous step S104, and thereafter the steps after step S104 are repeated.

### [Step S108 (preceding pump 1 a: FIG. 5)]

In step S108, in response to transmission of the message of starting cooperative administration to the succeeding pump 1 b side in the previous step S105, the control unit 21 of the preceding pump 1 a starts the cooperative administration of simultaneously driving the preceding pump 1 a and the succeeding pump 1 b after the cooperation start time Ts illustrated in FIG. 3. Then, the drive of the pump driving unit 11 is switched in accordance with the set sequence such that medicine administration is performed at the flow rate 100a set in the cooperation period Tab illustrated in FIG. 3, and cooperative administration of medicines is performed, and then, the cooperative administration is finished at the cooperation end time Tf.

### [Step S109 (preceding pump 1 a: FIG. 5)]

In step S109, when the cooperation period Tab illustrated in FIG. 3 is finished, the control unit 21 of the preceding pump 1 a causes the display unit 15 to display a warning of the end of medicine administration. Moreover, in a case where the preceding pump 1 a includes an alarm sound generator, it is allowable to cause the alarm sound to be generated.

### [Step S14 (preceding pump 1 a: FIG. 5)]

In step S14, a medical practitioner, an administrator, or the like, being qualified to operate the medical pump system 2 confirms the display of the end of medicine administration on the preceding pump 1a-side display unit 15, and then, performs an input of stopping the pump by operation on the operation unit 13. With this operation, continuous administration of the medicine on the side of the preceding pump 1 a is finished.

### [Step S205 (succeeding pump 1 b: FIG. 6)]

Meanwhile, upon determination of receiving (Yes) the signal of the cooperative administration start from the preceding pump 1 a (Yes) at the preceding step S204, the succeeding pump 1 b-side control unit 21 starts, in step S205, cooperative administration of simultaneously driving the preceding pump 1 a and the succeeding pump 1 b after the cooperation start time Ts illustrated in FIG. 3. Then, the drive of the pump driving unit 11 is switched in accordance with the set sequence such that medicine administration is performed at the flow rate 100b set in the cooperation period Tab illustrated in FIG. 3, and cooperative administration of medicines is performed, and then, the cooperative administration is finished at the cooperation end time Tf.

Note that the cooperative administration is started in synchronization with the start of cooperative administration in step S108 in the preceding pump 1 a illustrated in FIG. 5.

### [Step S206 (succeeding pump 1b: FIG. 6)]

Next, in step S206, the succeeding pump 1b-side control unit 21 starts single administration of medicine by driving the pump driving unit 11 such that the medicine is administered at the flow rate 100b set in the period Tb of the succeeding single administration illustrated in FIG. 3, and finishes the administration in accordance with the setting.

### [Step S207 (succeeding pump 1b: Fig. 6)]

In step S207, the control unit 21 of the succeeding pump 1b causes the display unit 15 to display a warning of the completion of medicine administration when the period Tb of the succeeding single administration illustrated in FIG. 3 has finished. Moreover, when the succeeding pump 1b includes an alarm sound generator, it is allowable to cause the alarm sound to be generated.

### [Step S22 (succeeding pump 1b: FIG. 6)]

Thereafter, in step S22, the medical practitioner, the administrator, or the like, being qualified to operate the medical pump system 2 confirms the display of the end of medicine administration on the succeeding pump 1 b-side display unit 15, and then, performs an input of stopping the pump by operation on the operation unit 13. With this operation, continuous administration of the medicine on the side of the succeeding pump 1 b is finished.

### <Effect of Embodiment>

In the above-described embodiment, in performing continuous administration of the medicine with application of the W method under the control of the control unit 21 of the preceding pump 1a, cooperative administration of simultaneously driving two medical pumps 1 can be arbitrarily started by an input on the operation unit 13 manually. Therefore, it is possible to efficiently monitor the switching operation of the pump in the cooperative administration period Tab by arbitrarily starting cooperative administration on the spot without waiting for the preset cooperation start time Ts.

Moreover, since the scheduled time until the cooperation start time Ts is configured to be displayed on the display unit 15, the administrator of the medical pump system 2 can easily determine whether to start cooperative administration without waiting for the cooperation start time Ts. Therefore, in a case where the scheduled time until the cooperation start time Ts is short, it is possible to make a determination of waiting until the cooperation start time Ts is reached without starting cooperative administration arbitrarily and then starting cooperative administration at the cooperation start time Ts following the setting while monitoring the switching operation of the pump. This makes it possible to monitor the switching operation of the pump in the cooperative administration period Tab without stress and to unnecessarily administer the medicine of the preceding pump 1a.

Note that the present invention is not limited to the embodiments and modifications described above and illustrated in the drawings, and various modifications can be made without departing from the scope and spirit of the invention described in the appended claims. For example, while in the above-described embodiment, the cooperative administration is started by the input from the preceding pump 1a side, it is also allowable to configure such that cooperative administration is started by the input from the succeeding pump 1b side.

Furthermore, continuous administration may be performed by replacing the succeeding pump 1b during the drive with the preceding pump 1a and using the additional medical pump 1 as the succeeding pump 1b. This continuous administration processing is started by operation, by the administrator of the medical pump system 2, of the operation unit 13 of the succeeding pump 1 b and the operation unit 13 of an additional medical pump 1 during single administration of the succeeding pump 1 b in step S206 of FIG. 6 (during the succeeding single period Tb illustrated in FIG. 3).

In this case, each of the control units 21 first establishes communication between the succeeding pump 1 b and the additional medical pump 1, and performs setting processing of replacing the succeeding pump 1 b with the preceding pump 1 a and setting the additional medical pump 1 to be the succeeding pump 1 b. Thereafter, processing with a procedure similar to the procedure described with reference to FIGS. 5 and 6 is performed on the newly set preceding pump 1 a and the succeeding pump 1 b. This makes it possible to continue continuous administration of the medicine with application of the W method by using the third and subsequent medical pumps 1.

In the above-described embodiment, the medical pump system 2 has been described having a configuration in which two medical pumps 1 can communicate with each other. Alternatively, the medical pump system according to the present invention may be configured such that two medical pumps 1 can mutually perform transmission and reception via a system control unit such as a host computer or via a communication unit of a pump stand to which a plurality of medical pumps 1 are attached. In this case, the above-described method for controlling the medical pump system is not limited to the configuration implemented by the control unit 21 of each of the medical pumps 1, but may be a configuration implemented by an instruction from a system control unit or from a communication unit connected to the communication unit of the pump stand.

### FIG. 1

11: PUMP DRIVING UNIT
13: OPERATION UNIT
15: DISPLAY UNIT
17: STORAGE UNIT
19: COMMUNICATION UNIT
21: CONTROL UNIT

### FIG. 3

FLOW RATE
FLOW RATE
TOTAL FLOW RATE
TIME

### FIG. 4

SCHEDULE TIME UNTIL COOPERATIVE ADMINISTRATION START
ABOUT 10 MINUTES
START COOPERATIVE ADMINISTRATION NOW?
YES
NO

### FIG. 5

START PROCESSING OF PRECEDING PUMP 1 a
S11: INPUT CONTINUOUS ADMINISTRATION SETTING
S12: INPUT ADMINISTRATION START
S13: INPUT COOPERATIVE ADMINISTRATION START
S14: INPUT PUMP STOP
S101: CONTINUOUS ADMINISTRATION SETTING PROCESSING FROM S202 OF SUCCEEDING PUMP 1 b
S102: COOPERATION AVAILABILITY DETERMINATION PROCESSING/TRANSMISSION/DISPLAY TO SUCCEEDING PUMP 1b
S103: START SINGLE ADMINISTRATION
S104: COOPERATION START TIME Ts?
S105: TRANSMISSION OF COOPERATIVE ADMINISTRATION START TO SUCCEEDING PUMP TO SUCCEEDING PUMP 1b
S106: DISPLAY SCHEDULED TIME UNTIL COOPERATION START TIME Ts AND DISPLAY
OPTIONS FOR COOPERATIVE ADMINISTRATION START
S107: IS COOPERATIVE ADMINISTRATION START SELECTED?
S108: COOPERATIVE ADMINISTRATION START/COOPERATIVE ADMINISTRATION
S109: DISPLAY WARNING OF END
END

### FIG. 6

START PROCESSING OF SUCCEEDING PUMP 1 b
S21: INPUT CONTINUOUS ADMINISTRATION SETTING
S22: INPUT PUMP STOP
S201: CONTINUOUS ADMINISTRATION SETTING PROCESSING
S202: TRANSMISSION OF MEDICINE INFORMATION TO PRECEDING PUMP 1 a TO PRECEDING PUMP 1 a
FROM S102 OF PRECEDING PUMP 1a
S203: START PROCESSING OF COOPERATIVE ADMINISTRATION
S204: HAS COOPERATION START BEEN RECEIVED?
S205: COOPERATIVE ADMINISTRATION START/COOPERATIVE ADMINISTRATION
S206: SINGLE ADMINISTRATION START/SINGLE ADMINISTRATION
S207: DISPLAY WARNING OF END
END

## Claims

1. A medical pump for achieving continuous administration of medicine by performing sequential driving with another medical pump, the medical pump comprising:
a pump driving unit for administering the medicine in accordance with a setting;
a communication unit for performing communication with the other medical pump;
a control unit for controlling individual driving of the pump driving unit and the communication unit; and
an operation unit connected to the control unit,
wherein, in a single administration period by a first medical pump in continuous administration of the medicine,
the control unit determines timing of starting cooperative administration of simultaneously driving the pump driving unit together with a pump driving unit of the other medical pump on the basis of the setting and on the basis of a manual input from the operation unit.

2. The medical pump according to claim 1, further comprising a display unit configured to display start information for starting the cooperative administration by the manual input from the operation unit during the single administration period.

3. The medical pump according to claim 1 or 2, further comprising a display unit configured to display a scheduled time based on the setting until the cooperative administration is started during the single administration period.

4. A medical pump system comprising:
two medical pumps for achieving continuous administration of medicine by sequential driving;
a control unit configured to control the continuous administration of the medicine by the two medical pumps; and
an operation unit connected to the control unit,
wherein each of the medical pumps includes:
a pump driving unit for administering the medicine in accordance with a setting; and
a communication unit for performing communication with the outside, and
the control unit determines, in a single administration period by a first medical pump among the two medical pumps, timing of starting cooperative administration of simultaneously driving the pump driving units of the two medical pumps on the basis of the setting and on the basis of a manual input from the operation unit.

5. A method for controlling a medical pump system configured to achieve continuous administration of medicine by sequentially driving two medical pumps on the basis of a setting,
wherein timing of starting cooperative administration of simultaneously driving the pump driving units of the two medical pumps during a single administration period of a first medical pump among the two medical pumps is determined in accordance with the setting and in accordance with an arbitrary input from the outside.
